# FASCICULE DE BREVET EUROPEEN

(11) **EP 2 111 849 B1**
(45) Date de publication et mention de la délivrance du brevet: **28.02.2018**
(21) Numéro de dépôt: 09158017.5
(22) Date de dépôt: 16.04.2009
(51) Int. Cl.: A61K 8/36, A61K 8/365, A61K 8/58, A61Q 5/02, A61Q 5/12

(54) **Utilisation d'une composition cosmétique comprenant au moins un composé organique du silicium en association avec une composition de soin et/ou de lavage des fibres kératiniques**
Anwendung einer kosmetischen Zusammensetzung, die mindestens eine organische Siliziumverbindung in Kombination mit einer Pflege-und/oder Reinigungszusammensetzung von Keratinfasern umfasst
Use of a cosmetic composition comprising at least one organic silicon compound associated with a composition for treating and/or washing keratinous fibres

(30) Priorité: 25.04.2008 FR 0852795
(43) Date de publication de la demande: 28.10.2009
(73) Titulaire: L'Oréal, 75008 Paris (FR)
(72) Inventeur: Woodland, Frédéric, 75018 Paris (FR); Lazzeri, Pascale, 13540 Puyricard (FR)
(74) Mandataire: Dossmann, Gérard

(56) Documents cités:
- WO-A-01/22925

## Description

La présente invention concerne l'utilisation, en association d'une composition de soin et/ou de lavage des fibres kératiniques, en particulier des fibres kératiniques humaines telles que les cheveux, et d'une composition cosmétique comprenant, dans un milieu cosmétiquement acceptable, un ou plusieurs composés organiques du silicium. La présente invention porte également sur un procédé de traitement des fibres kératiniques consistant à appliquer une composition cosmétique comprenant, dans un milieu cosmétiquement acceptable, un ou plusieurs composés organiques du silicium en association avec une composition de soin et/ou de lavage.

Les cheveux sont généralement abîmés et fragilisés par l'action des agents atmosphériques extérieurs tels que la lumière et les intempéries, et par des traitements mécaniques ou chimiques tels que le brossage, le peignage, les teintures, les décolorations, les permanentes et/ou les défrisages.

Ainsi pour remédier à ces inconvénients, il est maintenant usuel d'avoir recours à des soins capillaires impliquant la mise en oeuvre de produits de soins qui permettent de conditionner les cheveux, notamment en leur conférant de la douceur, de la brillance ainsi qu'un toucher naturel, et d'obtenir des effets coiffants.

Ces compositions de soins capillaires peuvent être notamment des shampooings conditionneurs ou des après-shampoings pouvant se présenter sous la forme de gels, de lotions capillaires ou de crèmes plus ou moins épaisses.

Par ailleurs, on a constaté que les consommateurs sont de plus en plus à la recherche de compositions de soins qui permettent non seulement de conditionner les cheveux de manière convenable mais également de procurer des effets coiffants satisfaisants.

En particulier, les personnes ayant des cheveux fins ou bouclés sont à la recherche de produits de soins qui procurent des effets coiffants apportant de la masse, du corps et du volume aux cheveux ainsi que du dessin à la boucle.

Toutefois, les compositions de soins usuelles procurent des effets coiffants qui sont relativement faibles et irréguliers, notamment en termes de dessins de boucle et de volume.

En effet, il est connu que l'introduction de composés organiques cosmétiquement actifs tels que des polymères cationiques et des silicones dans des compositions de soins telles que des après-shampooings, permet de conférer aux cheveux des propriétés de démêlage, de souplesse et de légèreté. Cependant, les propriétés coiffantes qui sont apportées aux cheveux restent encore nettement insuffisantes.

Il existe donc un réel besoin de mettre en oeuvre un procédé de traitement des cheveux, qui ne présente pas l'ensemble des inconvénients décrits ci-dessus, c'est-à-dire qui procure notamment des effets coiffants satisfaisants.

On connaît du document WO01/22925 A1 des compositions à base de composés organiques du silicium solubles dans l'eau, partiellement neutralisés, aptes à conférer aux cheveux un effet coiffant de longue durée et un toucher agréable. La demanderesse a découvert, de façon surprenante, qu'il était possible de mettre en oeuvre sur les fibres kératiniques, des compositions cosmétiques comprenant un ou plusieurs composés organiques du silicium et un ou plusieurs acides organiques carboxyliques, tels que définis ci-après, en association avec des compositions de soins et/ou de lavages des fibres kératiniques afin d'obtenir les propriétés recherchées.

Par "association", on entend au sens de la présente invention, que la composition cosmétique comprenant un ou plusieurs composés organiques du silicium et un ou plusieurs acides organiques carboxyliques, tels que définis ci-après, et la composition de soin et/ou de lavage sont appliquées sur les fibres kératiniques de manière séquentielle, c'est-à-dire, la composition cosmétique est appliquée en pré- ou post-traitement de la composition de soin et/ou de lavage. En effet, il a été constaté que l'utilisation d'une composition cosmétique comprenant de tels composés organiques du silicium et de tels acides organiques carboxyliques, en association avec des compositions de soins et/ou de lavages, telles que des shampooings ou des après-shampooings, permettait de conduire à un gainage satisfaisant des cheveux conférant ainsi des effets coiffants satisfaisants.

En particulier, les compositions conformes à l'invention utilisées en association avec des compositions de soins et/ou de lavages permettent de conférer du volume, du corps ainsi que du maintien à la coiffure notamment aux cheveux fins et d'apporter de la nervosité à la boucle de manière à obtenir des cheveux ayant des boucles mieux dessinées.

Les effets coiffants ainsi obtenus sont nettement plus marqués que ceux conférés par les compositions de soins et/ou de lavages utilisées seules.

De plus, les compositions utilisées en complément des compositions de soins et/ou de lavages permettent non seulement d'améliorer les effets coiffants mais également les propriétés cosmétiques conférées par les compositions de soins et/ou de lavages utilisées seules.

En effet, on a constaté que les compositions cosmétiques utilisées en complément des compositions de soins et/ou de lavages permettaient également d'améliorer la brillance, la souplesse ainsi que le lissage des cheveux par rapport aux compositions de soins et/ou de lavages utilisées seules.

Par ailleurs, dans le cas où l'on applique une composition comprenant des composés organiques du silicium en pré-traitement d'une composition de soin et/ou de lavage, on observe que les effets coiffants conférés par cette composition résistent à l'application d'un shampooing ou d'un après-shampooing. Ceci présente un avantage dans la mesure où l'utilisateur ne perd pas l'effet acquis par le pré-traitement lorsqu'il applique un shampooing ou un après-shampooing.

On a également constaté que les cheveux restaient mieux individualisés et pouvaient être démêlés plus facilement avec une composition cosmétique conforme à l'invention utilisée en association avec une composition de soin et/ou de lavage.

Par composition de soin, on entend au sens de la présente invention, une composition non lavante et de préférence n'altérant pas de manière significative la couleur et/ou l'intégrité des fibres kératiniques tout en améliorant l'aspect et/ou les propriétés de conditionnement desdites fibres. Ceci exclue les compositions de coloration, de permanente, c'est-à-dire les compositions réductrices et fixatrices (oxydantes), et de défrisage.

Au sens de la présente invention, les compositions de soins contiennent moins de 4% en poids de tensioactifs anioniques, de préférence moins de 2% en poids de tensioactifs anioniques, et plus particulièrement moins de 1 % en poids de tensioactifs anioniques par rapport au poids total de la composition de soin.

La présente invention concerne notamment l'utilisation d'une composition cosmétique comprenant, dans un milieu cosmétiquement acceptable, un ou plusieurs composés organiques du silicium, choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule, les silanes étant choisis parmi les composés de formule (I) telle que décrite ci-après, et un ou plusieurs acides organiques carboxyliques choisis parmi l'acide glycolique, l'acide lactique, l'acide tartrique et l'acide gluconique, en association avec une composition de soin et/ou de lavage des fibres kératiniques humaines, en particulier des fibres kératiniques humaines telles que les cheveux, ladite composition cosmétique et ladite composition de soin et/ou de lavage étant appliquées de manière séquentielle sur les fibres kératiniques.

En d'autres termes, la composition cosmétique comprenant, dans un milieu cosmétiquement acceptable, un ou plusieurs composés organiques du silicium et un ou plusieurs acides, tels que définis ci-avant, est utilisée en complément d'une composition de soin et/ou de lavage des fibres kératiniques.

Un autre objet de l'invention porte sur un procédé de traitement cosmétique des fibres kératiniques consistant à appliquer sur lesdites fibres kératiniques, de manière séquentielle, une composition cosmétique comprenant, dans un milieu cosmétiquement acceptable, un ou plusieurs composés organiques du silicium et un ou plusieurs acides, tels que décrits ci-avant, et une composition de soin et/ou de lavage, à rincer ou non après un éventuel temps de pose ou après un éventuel séchage.

D'autres objets et caractéristiques, aspects et avantages de l'invention apparaîtront encore plus clairement à la lecture de la description et des exemples qui suivent.

Les composés organiques du silicium utilisés dans la composition cosmétique selon l'invention sont choisis parmi les organosilanes, comprenant un atome de silicium et les organosiloxanes, comportant deux ou trois atomes de silicium, de préférence deux atomes de silicium. Ils doivent en outre comporter une ou plusieurs fonctions chimiques basiques, et de préférence une seule fonction chimique basique. La fonction chimique basique peut correspondre à toute fonction conférant un caractère basique au composé de silicium sans nuire à sa solubilité dans l'eau et est de préférence une fonction amine telle qu'une fonction amine primaire, secondaire ou tertiaire. La fonction chimique basique des composés du silicium selon l'invention, peut comporter éventuellement d'autres fonctions, telles que, par exemple, une autre fonction amine, une fonction acide ou une fonction halogène.

Le ou les composés organiques du silicium utilisés dans la composition cosmétique selon l'invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Le ou les organosilanes utilisés dans la composition cosmétique selon l'invention sont choisis parmi les composés de formule (I) : 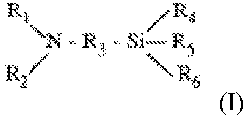 dans laquelle :
R₄ représente un halogène, un groupe OR' ou R'₁ ;
R₅ représente un halogène, un groupe OR" ou R'₂ ;
R₆ représente un halogène, un groupe OR''' ou R'₃ ;
R₁, R₂, R₃, R', R", R''', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R''' pouvant en outre désigner l'hydrogène, et deux au moins des groupes R₄, R₅ et R₆ désignant respectivement OR', OR" et OR''', deux au moins des groupes R', R" et R''' étant différents de l'hydrogène.

De préférence, les groupes R₁, R₂, R', R'₁, R'₂, R'₃, R" et R''' sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

Selon un autre mode de réalisation particulier, le ou les organosiloxanes sont choisis parmi les composés de formule (II) : 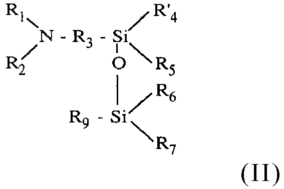 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈ , R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR''', OR₁₀ ou OR₈.

De préférence, les groupes R"₁, R"₂, R₈ ou R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

En particulier, l'atome d'halogène est un atome de chlore.

Le ou les composés organiques du silicium utilisés dans la composition cosmétique selon l'invention sont de préférence des organosilanes choisis parmi les composés de formule (III) : 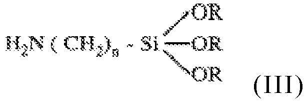 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium présent dans la composition cosmétique selon l'invention est le 3-aminopropyl triéthoxysilane.

Le ou les composés organiques du silicium peuvent être présents dans la composition cosmétique selon l'invention dans une teneur allant de 0,1 à 20 % en poids, de préférence dans une teneur en poids allant de 1 à 15 % en poids, et plus préférentiellement dans une teneur en poids allant de 2,5% à 12% en poids, par rapport au poids total de la composition cosmétique.

La composition cosmétique selon l'invention contient un ou plusieurs acides organiques carboxyliques choisis parmi l'acide glycolique, l'acide lactique, l'acide tartrique et l'acide gluconique. Plus préférentiellement, l'acide organique utilisé dans la composition selon l'invention est l'acide lactique.

Dans la composition, l'acide organique peut être sous forme libre ou salifiée.

Le ou les acides organiques utilisés dans la composition selon la présente invention peuvent être présents en une teneur exprimée en acides libres allant de 0,1 à 10% en poids, de préférence en une teneur allant de 0,5 à 8% en poids, et encore plus préférentiellement en une teneur allant de 1 à 5% en poids, par rapport au poids total de la composition.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

La composition cosmétique selon l'invention présente un pH allant de 3 à 11 et de préférence allant de 7 à 10.

La composition cosmétique selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ ; des céramides ; des esters gras tels que le myristate d'isopropyle, le myristate de myristyle, le palmitate de cétyle et le stéarate de stéaryle ; des huiles minérales, végétales ou synthétiques telles que les α-oléfines ou l'huile de palme ; des vitamines ou provitamines ; des polymères cationiques ou amphotères ; des silicones différents des composés organiques du silicium selon l'invention ; des agents de stabilisation du pH, des conservateurs ; et des colorants.

De préférence, la composition cosmétique selon l'invention comprenant un ou plusieurs agents épaississants.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition contenant les silanes et/ou les siloxanes de l'invention.

Les compositions cosmétiques selon l'invention peuvent se présenter sous diverses formes telles que des gels, des lotions ou des crèmes.

La composition cosmétique selon l'invention et la composition de soin et/ou de lavage sont appliquées de manière séquentielle sur les fibres kératiniques, en particulier les fibres kératiniques humaines telles que les cheveux, c'est-à-dire, la composition cosmétique selon l'invention est appliquée en pré- ou en post-traitement d'une composition de soin et/ou de lavage des fibres kératiniques. La composition cosmétique utilisée en pré- ou en post-traitement d'une composition de soin et/ou de lavage des fibres kératiniques peut être appliquée en mode rincé ou en mode non-rincé, c'est-à-dire que son application est suivie ou non d'un rinçage.

Encore plus préférentiellement, la composition cosmétique selon l'invention est utilisée en pré-traitement.

Les compositions de soins et/ou de lavages peuvent comprendre un ou plusieurs tensioactifs cationiques.

A titre d'exemples de tensioactif cationique, on peut notamment citer les sels d'amines grasses primaires, secondaires ou tertiaires, éventuellement polyoxyalkylénées et les sels d'ammonium quaternaire éventuellement polyoxyalkylénés.

De préférence, les tensioactifs cationiques sont choisis parmi les sels d'ammonium quaternaire éventuellement polyoxyalkylénés.

A titre de sels d'ammonium quaternaires, on peut notamment citer, par exemple :
- ceux qui présentent la formule générale (IV) suivante : 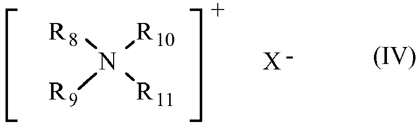 dans laquelle les radicaux R₁₂ à R₁₅, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes.

Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (V) suivante : 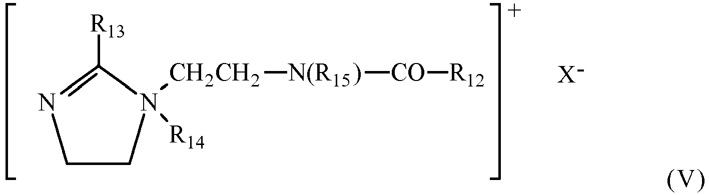 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (VI) : 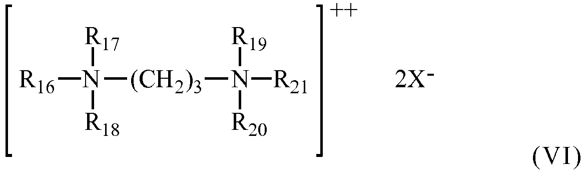 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (VII) suivante : 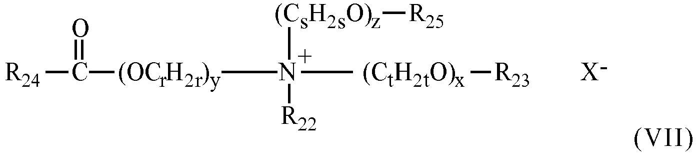 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
- le radical 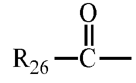
- les radicaux R₂₇ hydrocarbonés en C₁-C₂₂, linéaires ou ramifiés, saturés ou insaturés,
- l'atome d'hydrogène,
   R₂₅ est choisi parmi :
- le radical 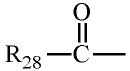
- les radicaux R₂₉ hydrocarbonés en C₁-C₆, linéaires ou ramifiés, saturés ou insaturés,
- l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6;
   y est un entier valant de 1 à 10 ;
   x et z, identiques ou différents, sont des entiers valant de 0 à 10 ;
   X⁻ est un anion simple ou complexe, organique ou inorganique;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (VII) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
- le radical 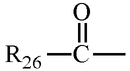
- les radicaux méthyle, éthyle ou hydrocarbonés en C₁₄-C₂₂,
- l'atome d'hydrogène ;
- R₂₅ est choisi parmi :
- le radical 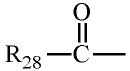
- l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition de soin et/ou de lavage peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant un e ou plusieurs fonctions esters décrits dans les brevets US-A-4874554 et US-A-4137180.

Parmi les sels d'ammonium quaternaire de formule (IV), on préfère d'une part, les chlorures de tétraalkylammonium comme, par exemple, les chlorures de dialkyldiméthylammonium ou d'alkyltrimé thylammonium ou alkylaralkyle diméthylammonium dans lesquels le radical alkyle comporte environ de 12 à 22 atomes de carbone, en particulier les chlorures de béhényltriméthyl ammonium, de distéaryldiméthylammonium, de cétyltriméthylammonium, de benzyldiméthylstéarylammonium ou encore, d'autre part, le chlorure de palmitamidopropyltriméthylammonium ou le chlorure de stéaramidopropyldiméthyl-(myristylacétate)-ammonium commercialisé sous la dénomination CERAPHYL® 70 par la société VAN DYK.

De préférence, le tensioactif cationique utilisé dans la composition de soin et/ou de lavage est choisi parmi le chlorure de béhényltriméthyl ammonium et de cétyltriméthyl ammonium et leurs mélanges.

Le ou les tensioactifs cationiques est ou sont présents en une quantité allant de 0,1 à 6% en poids de tensioactifs cationiques, de préférence en une quantité variant de 0,5 à 3% en poids, par rapport au poids total de la composition de soin et/ou de lavage, et de préférence par rapport au poids total de la composition de soin.

Les compositions de soins et/ou de lavages peuvent comprendre également un ou plusieurs tensioactifs choisis parmi les tensioactifs anioniques, amphotères et non ioniques.

De préférence, les compositions de lavages comprennent un ou plusieurs tensioactifs anioniques.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Le ou les tensioactifs anioniques peuvent être présents dans une teneur d'au moins 4 % en poids, par rapport au poids total de la composition de lavage.

Le ou les tensioactifs anioniques peuvent être notamment présents dans une teneur allant de 0,01 à 50% en poids et, de préférence, allant de 0,1 à 20% en poids par rapport au poids total de la composition de soin et/ou de lavage.

De préférence, la composition de soin contient moins de 4% en poids de tensioactifs anioniques.

Encore plus préférentiellement, la composition de soin ne contient pas de tensioactifs anioniques.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels est de préférence comprise dans l'intervalle allant de 0,01 à 20 % en poids, mieux encore de 0,1 à 10 % en poids par rapport au poids total de la composition de soin et/ou de lavage.

Les agents tensioactifs amphotères ou zwittérioniques, utilisables dans la composition de soin et/ou de lavage, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH₂CH₂-N(B)(B') (B)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,01, à 20%, mieux encore de 0,1 à 10 % en poids par rapport au poids total de la composition de soin et/ou de lavage.

Les compositions de soins et/ou de lavages peuvent être utilisées pour le lavage et le conditionnement des fibres kératiniques, en particulier des cheveux, par exemple comme shampooings, de préférence comme shampooings conditionneurs, ou encore pour le conditionnement des fibres kératiniques, par exemple comme après shampooings.

Dans le cas des shampooings conditionneurs, la composition contient un ou plusieurs tensioactifs anioniques.

De préférence, les compositions de soin sont des avant- ou après-shampooings.

De préférence, les compositions de soins et/ou de lavages sont des après-shampooings à rincer ou non contenant de préférence un ou plusieurs tensioactifs cationiques.

Les compositions de soins et/ou de lavages peuvent comprendre en outre un ou plusieurs additifs classiques bien connus dans la technique, tels que des épaississants ou régulateurs de viscosité, naturels ou synthétiques ; des céramides ; des silicones différentes des composés organiques du silicium utilisés dans la composition de pré- ou de post-traitement ; des esters gras huileux tels que le myristate d'isopropyle; des huiles minérales, végétales ou synthétiques telles que les α-oléfines ; des vitamines ou provitamines ; des polymères cationiques ou amphotères; des agents de stabilisation du pH, des conservateurs ; et des colorants.

La présente invention a aussi pour objet un procédé de traitement cosmétique des fibres kératiniques, en particulier des fibres kératiniques humaines telles que les cheveux, qui consiste à appliquer de manière séquentielle sur lesdites fibres kératiniques, une composition cosmétique comprenant, dans un milieu cosmétiquement acceptable, un ou plusieurs composés organiques du silicium tels que définis ci-avant et une composition de soin et/ou de lavage, à rincer ou non après un éventuel temps de pose ou après un éventuel séchage.

Ce procédé de traitement peut être mis en oeuvre sur des fibres kératiniques naturelles ou des fibres qui ont subi un traitement cosmétique tel qu'une permanente, une coloration, une décoloration ou un défrisage.

En particulier, le procédé de traitement consiste à appliquer sur lesdites fibres kératiniques, une composition cosmétique de pré-traitement comprenant, dans un milieu cosmétiquement acceptable, un ou plusieurs composés organiques du silicium tels que définis ci-avant, à rincer ou non après un éventuel temps de pose ou après un éventuel séchage, puis à appliquer une composition de soin et/ou de lavage, à rincer ou non après un éventuel temps de pose ou après un éventuel séchage.

Dans le cas de ce mode réalisation, la composition de soin et/ou de lavage est de préférence rincée.

Selon une variante, le procédé de traitement consiste à appliquer sur lesdites fibres kératiniques, une composition de soin et/ou de lavage, à rincer ou non après un éventuel temps de pose ou après un éventuel séchage, puis à appliquer une composition cosmétique de post-traitement comprenant, dans un milieu cosmétiquement acceptable, un ou plusieurs composés organiques du silicium tels que définis ci-avant, à rincer ou non après un éventuel temps de pose ou après un éventuel séchage.

De préférence, les compositions de pré-traitement ne sont pas rincées, c'est-à-dire que leur application n'est pas suivie d'un rinçage.

Comme indiqué précédemment, le pré-traitement avec la composition contenant le ou les composés organiques du silicium est préféré.

Le temps de pose de la composition de pré- ou de post-traitement et de la composition de soin et/ou de lavage peut être compris entre quelques secondes et 60 minutes et de préférence entre 30 secondes et 15 minutes.

Il est également possible d'appliquer la composition de pré- ou de post-traitement et la composition de soin et/ou de lavage à plusieurs reprises.

Dans tous les cas, l'étape de séchage éventuellement présente peut être effectuée au casque, au sèche cheveux et/ou au fer à lisser.

Les exemples qui suivent servent à illustrer l'invention sans toutefois présenter un caractère limitatif.

### EXEMPLE I

Dans l'exemple suivant, on a évalué les effets coiffants obtenus sur cheveux fins et sur cheveux bouclés avec une composition conforme à l'invention qui est utilisée en pré-traitement d'un après-shampooing.

### 1. Composition testée

On prépare une composition A à partir des ingrédients indiqués dans le tableau ci-dessous dont les quantités sont exprimées en pour cent en poids, par rapport au poids total de la composition.

| Composition A | |
|---|---|
| 3-aminopropyltriéthoxysilane | 10 |
| Acide lactique | 4 |
| Eau | qsp 100 |

### 2. Mode opératoire

20 modèles (10 cheveux fins et 10 cheveux bouclés) ont appliqué la composition A sur leurs cheveux. Après 30 secondes de pause sans rinçage intermédiaire, les modèles ont appliqué un après-shampoing ULTRADOUX® CERISE ET NACRE comprenant à titre d'actif 0,8 % MA de chlorure de cétyl triméthylammonium, 1% en poids de Quaternium 80 et 1,35% en poids de Dipalmitoyléthylhydroxyéthylammonium méthosulfate. Après 30 secondes, les cheveux sont rincés et séchés.

### 3. Résultats

Les 10 testeurs aux cheveux fins ont jugé unanimement que les cheveux sont plus volumineux que dans le cadre de l'utilisation d'un après shampooing sans utilisation du pré-traitement.

Sur les 10 testeurs aux cheveux bouclés, 7 sur 10 ont trouvé que les boucles de cheveux sont mieux dessinées et 8 sur 10 ont troouvé les boucles de cheveux plus nerveuses.

### EXEMPLE II

Dans l'exemple suivant, on a évalué les effets coiffants obtenus sur cheveux fins et sur cheveux bouclés avec une composition conforme à l'invention qui est utilisée en pré-traitement d'un shampooing.

### 1. Composition testée

On prépare une composition B et une composition C à partir des ingrédients indiqués dans le tableau ci-dessous dont les quantités sont exprimées en pour cent en poids, par rapport au poids total de la composition.

| | Composition B | Composition C |
|---|---|---|
| 3-aminopropyltriéthoxysilane ⁽¹⁾ | 10 | - |
| Acide lactique | 4 | - |
| Hydroxyéthylcellulose | 0,7 | 0,7 |
| Hydroxyde de potassium | - | Qs pH composition B = 9 |
| Parfum, colorants | Qs | Qs |
| Eau | qsp 100 | qsp 100 |

La composition B présente une valeur de pH égale à 9.

### 2. Mode opératoire

Sur six modèles sont appliqués en comparatif d'un côté de la tête 6g de la composition B et de l'autre 6g de la composition C. Après 30 secondes de pause et sans rinçage intermédiaire sont appliqués de part et d'autre 6g de shampooing Elsève Multivitamines 2-en-1 qui contient 15,5% de lauryléthylsulfate de sodium et 2,4% en poids de cocobétaïne. Le shampooing est ensuite rincé et les cheveux séchés.

### 3. Résultats

Un panel d'experts effectue ensuite une évaluation comparative en attribuant une note allant de 1 à 5. Les moyennes de ces notes sont regroupées dans le tableau ci-dessous.

| | Evaluation sensorielle | Composition B + Shampooing | Composition C + Shampooing |
|---|---|---|---|
| Lors du rinçage | Lissage | 3,3 | 2,4 |
| | Souplesse | 3,4 | 2,6 |
| Sur Cheveux humides | Démêlage | 4,1 | 3,3 |
| | Lissage | 3,3 | 2,8 |

Ces résultats montrent que, sur cheveux humides, les cheveux sont plus lisses et se démêlent plus facilement avec la composition B, conforme à l'invention, utilisée en pré-traitement d'un shampooing qu'avec une composition C, non conforme à l'invention, utilisée en pré-traitement d'un shampooing.

Ces résultats montrent également que lors du rinçage du shampooing les cheveux sont plus lisses et plus souples avec la composition B, conforme à l'invention qui est utilisée en pré-traitement d'un shampooing, qu'avec une composition C, non conforme à l'invention, utilisée en pré-traitement d'un shampooing.

### EXEMPLE III

Dans l'exemple suivant, on a comparé les effets coiffants et les propriétés cosmétiques obtenus avec une composition utilisée en pré-traitement d'un après-shampooing à ceux obtenus avec un après-shampooing dans lequel le prétraitement a été remplacé par un simple traitement avec de l'eau.

### 1. Composition testée

On prépare une composition A identique à celle décrite dans l'exemple I.

### 2. Modes opératoires

Sur six modèles dont la chevelure à été lavée sont appliqués en comparatif d'un côté de la tête 6g de la composition A et de l'autre 6g d'eau. Après 30 secondes de pause et sans rinçage intermédiaire sont appliqués de part et d'autre 6g d'après-shampooing L'Oréal Professionnel Lumi Care qui contient 0,8 % MA de chlorure de cétyl triméthylammonium, 1% en poids de Quaternium 80 et 1,35% en poids de Dipalmitoyléthylhydroxyéthylammonium méthosulfate. L'après-shampooing est ensuite rincé et les cheveux séchés. Cette application est répétée trois fois de suite à 48h d'intervalle sur une période de 5 jours. Les évaluations sont faites lors de cette troisième application.

### 3. Résultats

Un panel d'experts effectue ensuite une évaluation comparative en attribuant une note allant de 1 à 5. Les moyennes de ces notes sont regroupées dans le tableau ci-dessous.

| | Evaluation sensorielle | Composition A + Après shampooing | Eau + Après shampooing |
|---|---|---|---|
| Cheveux humides | Souplesse | 3,1 | 2,4 |
| Cheveux secs | Brillance | 3,7 | 3,3 |
| | Lissage visuel | 3,4 | 2,8 |
| | Lissage toucher | 3,9 | 3,3 |

Ces résultats montrent que, sur cheveux humides, les cheveux sont plus souples avec la composition A utilisée en pré-traitement d'un après-shampooing qu'avec un simple prétraitement avec de l'eau.

Ces résultats montrent aussi que, sur cheveux secs, les cheveux sont plus brillants, plus lisses au toucher ainsi que visuellement avec la composition A utilisée en pré-traitement d'un après-shampooing qu'avec un simple prétraitement avec de l'eau.

En outre, on a constaté que les cheveux sont enrobés de façon plus homogènes ce qui confère plus de volume aux cheveux.

### EXEMPLE IV

Dans l'exemple suivant, on a comparé les effets coiffants et les propriétés cosmétiques obtenus avec une composition utilisée en post-traitement d'un après-shampooing conformément à l'invention à ceux obtenus avec un après-shampooing utilisé avec un post traitement constitué uniquement par de l'eau.

### 1. Composition testée

### On prépare une composition A identique à celle décrite dans l'exemple I

### 2. Modes opératoires

Sur six modèles dont la chevelure à été lavée sont appliqués de part et d'autre 6g d'après-shampooing L'Oréal Professionnel Lumi Care qui contient 0,8 % MA de chlorure de cétyl triméthylammonium, 1% en poids de Quaternium 80 et 1,35% en poids de Dipalmitoyléthylhydroxyéthylammonium méthosulfate. Après 30 secondes de pause et sans rinçage intermédiaire sont appliqués en comparatif d'un côté de la tête 6g de la composition A et de l'autre 6g d'eau. Les cheveux sont ensuite rincés puis séchés. Cette application est répétée trois fois de suite à 48h d'intervalle sur une période de 5 jours. Les évaluations sont faites lors de cette troisième application.

### 3. Résultats

Un panel d'experts effectue ensuite une évaluation comparative en attribuant sur certains critères une note allant de 1 à 5. Les moyennes de ces notes sont regroupées dans le tableau ci-dessous.

| | Evaluation sensorielle | Après shampooing + Composition A | Après shampooing + Eau |
|---|---|---|---|
| Cheveux humides | Individualisation | 2,5 | 1,8 |
| | Souplesse | 3,5 | 2,9 |
| | Lisse au toucher | 3,2 | 2,5 |
| Cheveux secs | Individualisation | 2,7 | 2,1 |

Ces résultats montrent que, sur cheveux humides, les cheveux sont plus individualisés et mieux enrobés avec la composition A utilisée en post-traitement d'un après-shampooing qu'avec un simple post traitement avec de l'eau.

Ces résultats montrent également que, sur cheveux secs, les cheveux sont plus souples, plus lisses au toucher, plus individualisés avec la composition A utilisée en post-traitement d'un après-shampooing qu'en l'absence de ce post-traitement.

En outre, on a constaté que la composition A utilisée en post-traitement d'un après-shampooing confère plus de volume aux cheveux qu'avec un simple post traitement avec de l'eau.

## Revendications

1. Utilisation d'une composition cosmétique comprenant, dans un milieu cosmétiquement acceptable, un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule, les silanes étant choisis parmi les composés de formule (I) : 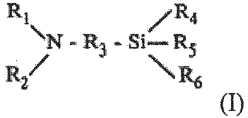 dans laquelle :
R₄ représente un halogène, un groupe OR' ou R'₁ ;
R₅ représente un halogène, un groupe OR" ou R'₂ ;
R₆ représente un halogène, un groupe OR''' ou R'₃ ;
R₁, R₂, R₃, R', R", R''', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R''' pouvant en outre désigner l'hydrogène, et deux au moins des groupes R₄, R₅ et R₆ désignant respectivement OR', OR" et OR''', deux au moins des groupes R', R" et R'" étant différents de l'hydrogène, et un ou plusieurs acides organiques carboxyliques choisis parmi l'acide glycolique, l'acide lactique, l'acide tartrique, l'acide gluconique,
en association avec une composition de soin et/ou de lavage des fibres kératiniques, en particulier des fibres kératiniques humaines telles que les cheveux ; ladite composition cosmétique étant appliquée en pré- ou post-traitement de ladite composition de soin et/ou de lavage sur les fibres kératiniques.

2. Utilisation selon la revendication 1, **caractérisée en ce que** les fonctions chimiques basiques du composé organique du silicium sont choisies parmi les amines primaires, secondaires ou tertiaires.

3. Utilisation selon la revendication 1 ou 2, **caractérisée en ce que** les groupes hydrolysables sont choisis parmi les groupes alcoxy, aryloxy et halogène.

4. Utilisation selon l'une quelconque des revendications 1 à 3, **caractérisée en ce que** le ou les siloxanes sont choisis parmi les composés de formule (II) : 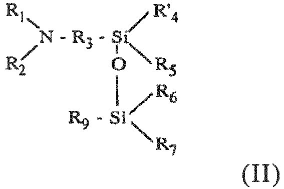 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR''', OR₁₀ ou OR₈.

5. Utilisation selon la revendication 1 ou 4, **caractérisée en ce que** les groupes R₁ R₂, R', R'₁, R'₂, R'₃, R", R''', R"₁, R"2, R₈, Rio et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

6. Utilisation selon l'une quelconque des revendications précédentes, **caractérisée en ce que** le ou les composés organiques du silicium sont choisis parmi les composés de formule (III) : 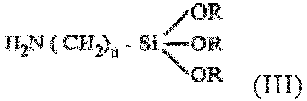 dans laquelle les radicaux R, identiques ou différents, sont choisis parmi les radicaux alkyle en C₁-C₆, de préférence C₁-C₂, et n est un nombre entier de 1 à 6, de préférence de 2 à 4.

7. Utilisation selon l'une quelconque des revendications précédentes, **caractérisée en ce que** le ou les composés organiques du silicium sont présents dans une teneur allant de 0,1 à 20 % en poids, de préférence dans une teneur en poids allant de 1 à 15 % en poids, et plus préférentiellement dans une teneur en poids allant de 2,5 à 12% en poids, par rapport au poids total de la composition.

8. Utilisation selon quelconque des revendications précédentes, **caractérisée en ce que** le ou les acides organiques sont présents en une teneur allant de 0,1 à 10% en poids, de préférence en une teneur allant de 0,5 à 8% en poids, et encore plus préférentiellement en une teneur allant de 1 à 5% en poids, par rapport au poids total de la composition cosmétique.

9. Utilisation selon l'une quelconque des revendications précédentes, **caractérisée en ce que** la composition de soin et/ou de lavage est un shampooing ou un après-shampooing.

10. Procédé de traitement cosmétique des fibres kératiniques, **caractérisé en ce qu'**il consiste à appliquer sur les fibres kératiniques, une composition cosmétique de pré-traitement comprenant, dans un milieu cosmétiquement acceptable, un ou plusieurs composés organiques du silicium tels que définis selon l'une quelconque des revendications 1 à 6 et un ou plusieurs acides organiques carboxyliques choisis parmi l'acide glycolique, l'acide lactique, l'acide tartrique, l'acide gluconique, rincer ou non après un éventuel temps de pose ou après un éventuel séchage, puis à appliquer une composition de soin et/ou de lavage, à rincer ou non après un éventuel temps de pose ou après un éventuel séchage.

11. Procédé de traitement cosmétique des fibres kératiniques, **caractérisé en ce qu'**il consiste à appliquer sur les fibres kératiniques, une composition de soin et/ou de lavage, à rincer ou non après un éventuel temps de pose ou après un éventuel séchage, puis à appliquer une composition cosmétique de post-traitement comprenant, dans un milieu cosmétiquement acceptable, un ou plusieurs composés organiques du silicium tels que définis selon l'une quelconque des revendications 1 à 6 et un ou plusieurs acides organiques carboxyliques choisis parmi l'acide glyconique, l'acide lactique, l'acide tartrique, l'acide gluconique, à rincer ou non après un éventuel temps de pose ou après un éventuel séchage.

## Patentansprüche

1. Verwendung einer kosmetischen Zusammensetzung, umfassend, in einem kosmetisch annehmbaren Medium, eine oder mehrere organische Siliziumverbindungen, ausgewählt aus Silanen, umfassend ein Siliziumatom und Siloxane, umfassend zwei oder drei Siliziumatome, wobei die organischen Siliziumverbindungen ferner eine oder mehrere basische chemische Funktionen und eine oder mehrere Hydroxylgruppen oder hydrolysierbare Gruppen pro Molekül umfassen, wobei die Silane ausgewählt sind aus den Verbindungen der Formel (I) : 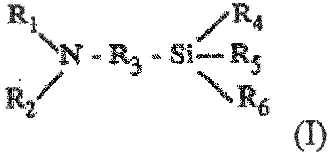 worin:
R₄ für ein Halogen, eine OR'- oder R'₁-Gruppe steht;
R₅ für ein Halogen, eine OR" - oder R'₂-Gruppe steht;
R₆ für ein Halogen, eine OR'''- oder R'₃-Gruppe steht;
R₁, R₂, R₃, R', R", R''', R'₁, R'₂, R'₃ unabhängig voneinander für eine lineare oder verzweigte, gesättigte oder ungesättigte Kohlenwasserstoffgruppe stehen, die gegebenenfalls zusätzliche chemische Gruppen trägt, wobei R₁, R₂, R', R" und R''' ferner Wasserstoff bezeichnen können, und mindestens zwei der Gruppen R₄, R₅ und R₆ OR', OR" bzw. OR''' bezeichnen, wobei mindestens zwei der Gruppen R', R" und R''' verschieden von Wasserstoff sind, und eine oder mehrere organische Carbonsäuren, ausgewählt aus Glycolsäure, Milchsäure, Weinsäure, Gluconsäure, in Kombination mit einer Zusammensetzung zum Pflegen und/oder zum Reinigen von Keratinfasern, insbesondere menschlichen Keratinfasern, wie beispielsweise Haaren; wobei die kosmetische Zusammensetzung als Vor- oder Nachbehandlung der Zusammensetzung zum Pflegen und/oder zum Reinigen auf die Keratinfasern aufgetragen wird.

2. Verwendung nach Anspruch 1, **dadurch gekennzeichnet, dass** die basischen chemischen Funktionen der organischen Siliziumverbindung ausgewählt sind aus den primären, sekundären oder tertiären Aminen.

3. Verwendung nach Anspruch 1 oder 2, **dadurch gekennzeichnet, dass** die hydrolysierbaren Gruppen ausgewählt sind aus den Alkoxy-, Aryloxy- und Halogengruppen.

4. Verwendung nach einem der vorhergehenden Ansprüche 1 bis 3, **dadurch gekennzeichnet, dass** das oder die Siloxane ausgewählt sind aus den Verbindungen der Formel (II): 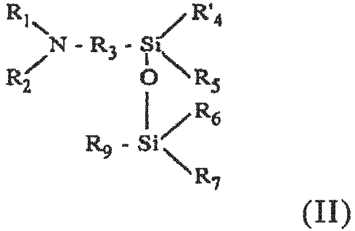 worin:
R₁, R₂, R₃, R₅ und R₆ wie oben definiert sind;
R'₄ für ein Halogenatom oder eine OR₁₁-Gruppe steht;
R₇ für ein Halogenatom, eine OR₁₀- oder R"₁-Gruppe steht;
R₉ für ein Halogenatom, eine OR₈-, R"₂- oder R₃NR₁R₂-Gruppe steht;
R"₁, R"₂, R₈, R₁₀ und R₁₁ für eine lineare oder verzweigte, gesättigte oder ungesättigte Kohlenwasserstoffgruppe stehen, die gegebenenfalls zusätzliche chemische Gruppen trägt, wobei die Gruppen R₁₁, R₁₀ und R₈ ferner ein Wasserstoffatom darstellen können; wobei mindestens eine der Gruppen R₆, R₇ und R₉ ein Halogenatom, eine OR'''-, OR₁₀- oder OR₈-Gruppe bezeichnet.

5. Verwendung nach Anspruch 1 oder 4, **dadurch gekennzeichnet, dass** die Gruppen R₁, R₂, R', R'₁, R'₂, R'₃, R", R''', R"₁, R"₂, R₈, R₁₀ und R₁₁ ausgewählt sind aus C₁-C₁₂-Alkyl-, C₆- bis C₁₄-Aryl-, C₁- bis C₈-Aryl-C₆-bis C₁₄-Alkyl- und C₆-bis C₁₄-Alkyl-C₁-bis C₈-Arylradikalen.

6. Verwendung nach einem der vorhergehenden Ansprüche, **dadurch gekennzeichnet, dass** die organische(n) Siliziumverbindung(en) ausgewählt ist/sind aus den Verbindungen der Formel (III): 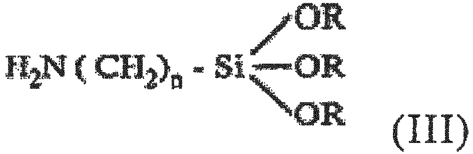 worin die Radikale R, gleich oder verschieden, ausgewählt sind aus den C₁-C₆-, vorzugsweise C₁-C₂-Alkylradikalen, und n eine ganze Zahl von 1 bis 6, vorzugsweise von 2 bis 4 ist.

7. Verwendung nach einem der vorhergehenden Ansprüche, **dadurch gekennzeichnet, dass** die organische(n) Siliziumverbindung(en) in einem Anteil im Bereich von 0,1 bis 20 Gew.-%, vorzugsweise in einem Gewichtsanteil im Bereich von 1 bis 15 Gew.-% und stärker bevorzugt in einem Gewichtsanteil im Bereich von 2,5 bis 12 Gew.-%, bezogen auf das Gesamtgewicht der Zusammensetzung, vorliegt/vorliegen.

8. Verwendung nach einem der vorhergehenden Ansprüche, **dadurch gekennzeichnet, dass** die organische(n) Säure(n) in einem Anteil im Bereich von 0,1 bis 10 Gew.-%, vorzugsweise in einem Anteil im Bereich von 0,5 bis 8 Gew.-% und noch stärker bevorzugt in einem Anteil im Bereich von 1 bis 5 Gew.-%, bezogen auf das Gesamtgewicht der kosmetischen Zusammensetzung, vorliegt/vorliegen.

9. Verwendung nach einem der vorhergehenden Ansprüche, **dadurch gekennzeichnet, dass** es sich bei der Zusammensetzung zum Pflegen und/oder zum Reinigen um ein Shampoo oder um einen Conditioner handelt.

10. Verfahren zur kosmetischen Behandlung von Keratinfasern, **dadurch gekennzeichnet, dass** es aus Auftragen einer kosmetischen Zusammensetzung zur Vorbehandlung, umfassend, in einem kosmetisch annehmbaren Medium, eine oder mehrere organische Siliziumverbindungen, wie nach einem der Ansprüche 1 bis 6 definiert, und eine oder mehrere organische Carbonsäuren, ausgewählt aus Glykolsäure, Milchsäure, Weinsäure, Gluconsäure, die nach einer optionalen Einwirkzeit oder nach einem optionalen Trocknen ausgespült wird oder nicht, auf die Keratinfasern, dann Auftragen einer Zusammensetzung zum Pflegen und/oder zum Reinigen besteht, die nach einer optionalen Einwirkzeit oder nach einem optionalen Trocknen ausgespült wird oder nicht.

11. Verfahren zur kosmetischen Behandlung von Keratinfasern, **dadurch gekennzeichnet, dass** es aus Auftragen einer kosmetischen Zusammensetzung zum Pflegen und/oder zum Reinigen, die nach einer optionalen Einwirkzeit oder nach einem optionalen Trocknen ausgespült wird oder nicht, auf die Keratinfasern, dann Auftragen einer kosmetischen Zusammensetzung zur Nachbehandlung besteht, umfassend, in einem kosmetisch annehmbaren Medium, eine oder mehrere organische Siliziumverbindungen, wie nach einem der Ansprüche 1 bis 6 definiert, und eine oder mehrere organische Carbonsäuren, ausgewählt aus Glykolsäure, Milchsäure, Weinsäure, Gluconsäure, die nach einer optionalen Einwirkzeit oder nach einem optionalen Trocknen ausgespült wird oder nicht.

## Claims

1. Use of a cosmetic composition comprising, in a cosmetically acceptable medium, one or more organic silicon compounds chosen from silanes comprising one silicon atom and siloxanes comprising two or three silicon atoms, the said organic silicon compounds additionally comprising one or more basic chemical functional groups and one or more hydroxyl or hydrolysable groups per molecule, the silanes being chosen from the compounds of formula (I): 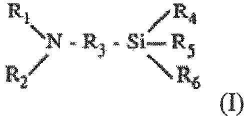 in which:
R₄ represents a halogen or an OR' or R'₁ group;
R₅ represents a halogen or an OR" or R'₂ group;
R₆ represents a halogen or an OR''' or R'₃ group;
R₁, R₂, R₃, R', R", R''', R'₁, R'₂ and R'₃ represent, independently of one another, a saturated or unsaturated and linear or branched hydrocarbon group optionally carrying additional chemical groups, R₁, R₂, R', R" and R''' being able in addition to denote hydrogen and two at least of the groups R₄, R₅ and R₆ respectively denoting OR', OR" and OR''', two at least of the groups R', R" and R''' being other than hydrogen, and one or more organic carboxylic acids chosen from glycolic acid, lactic acid, tartaric acid and gluconic acid,
in combination with a composition for caring for and/or washing keratinous fibres, in particular human keratinous fibres, such as the hair; said cosmetic composition being applied as pre- or as post-treatment for said care and/or washing composition to the keratinous fibres.

2. Use according to Claim 1, **characterized in that** the basic chemical functional groups of the organic silicon compound are chosen from primary, secondary or tertiary amines.

3. Use according to Claim 1 or 2, **characterized in that** the hydrolysable groups are chosen from alkoxy, aryloxy and halogen groups.

4. Use according to any one of Claims 1 to 3, **characterized in that** the siloxane or siloxanes are chosen from the compounds of formula (II): 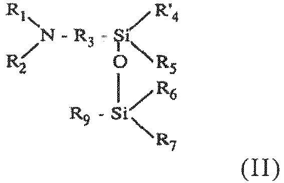 in which:
R₁, R₂ , R₃ , R₅ and R₆ are defined as above;
R'₄ represents a halogen atom or an OR₁₁ group;
R₇ represents a halogen atom or an OR₁₀ or R"₁ group;
R₉ represents a halogen atom or an OR₈, R"₂ or R₃NR₁R₂ group;
R"₁, R"₂, R₈, R₁₀ and R₁₁ represent a saturated or unsaturated and linear or branched hydrocarbon group optionally carrying additional chemical groups, the groups R₁₁, R₁₀ and R₈ being able in addition to represent a hydrogen atom; one at least of the groups R₆, R₇ and R₉ denoting a halogen atom or an OR''', OR₁₀ or OR₈ group.

5. Use according to Claim 1 or 4, **characterized in that** the R₁, R₂, R', R'₁, R'₂, R'₃, R", R''', R"₁, R"₂, R₈, R₁₀ and R₁₁ groups are chosen from C₁-C₁₂ alkyl, C₆ to C₁₄ aryl, (C₁ to C₈)alkyl (C₆ to C₁₄)aryl and (C₆ to C₁₄)aryl(C₁ to C₈)alkyl radicals.

6. Use according to any one of the preceding claims, **characterized in that** the organic silicon compound or compounds are chosen from the compounds of formula (III): 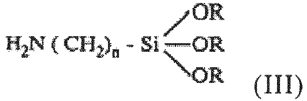 in which the R radicals, which are identical or different, are chosen from C₁-C₆, preferably C₁-C₂, alkyl radicals and n is an integer from 1 to 6, preferably from 2 to 4.

7. Use according to any one of the preceding claims, **characterized in that** the organic silicon compound or compounds are present in a content ranging from 0.1 to 20% by weight, preferably in a content ranging from 1 to 15% to weight and more preferably in a content ranging from 2.5 to 12% by weight, with respect to the total weight of the composition.

8. Use according to any one of the preceding claims, **characterized in that** the organic acid or acids are present in a content ranging from 0.1 to 10% by weight, preferably in a content ranging from 0.5 to 8% by weight and more preferably still in a content ranging from 1 to 5% by weight, with respect to the total weight of the cosmetic composition.

9. Use according to any one of the preceding claims, **characterized in that** the care and/or washing composition is a shampoo or a conditioner.

10. Method for the cosmetic treatment of keratinous fibres, **characterized in that** it consists in applying, to the keratinous fibres, a pretreatment cosmetic composition comprising, in a cosmetically acceptable medium, one or more organic silicon compounds as defined according to any one of Claims 1 to 6, and one or more organic carboxylic acids chosen from glycolic acid, lactic acid, tartaric acid and gluconic acid, in rinsing or in not rinsing out after an optional leave-in time or after an optional drying, in then applying a care and/or washing composition and in rinsing or in not rinsing out after an optional leave-in time or after an optional drying.

11. Method for the cosmetic treatment of keratinous fibres, **characterized in that** it consists in applying, to the keratinous fibres, a care and/or washing composition, in rinsing or in not rinsing out after an optional leave-in time or after an optional drying, in then applying a post-treatment cosmetic composition comprising, in a cosmetically acceptable medium, one or more organic silicon compounds as defined according to any one of Claims 1 to 6 and one or more organic carboxylic acids chosen from glycolic acid, lactic acid, tartaric acid and gluconic acid, and in rinsing or in not rinsing out after an optional leave-in time or after an optional drying.
